# EUROPEAN PATENT SPECIFICATION

(11) **EP 4 504 141 B1**
(45) Date of publication and mention of the grant of the patent: **29.07.2026**
(21) Application number: 23711744.5
(22) Date of filing: 17.03.2023
(51) Int. Cl.: A61K 8/73, A61Q 11/00, A61K 8/34

(54) **A NON AQUEOUS ORAL CARE COMPOSITION BASED ON POLYOL AND CARRAGEENAN**
NICHT NICHTWÄSSRIGE MUNDPFLEGEZUSAMMENSETZUNG AUF BASIS VOM POLYOL UND CARRAGEEN
COMPOSITION DE SOIN BUCCAL COMPRENANT UN POLYOL ET DU CARRAGEENAN

(30) Priority: 01.04.2022 EP 22166287
(43) Date of publication of application: 12.02.2025
(73) Proprietor: Unilever IP Holdings B.V., 6708 WH Wageningen (NL); Unilever Global IP Limited, Wirral, Merseyside CH62 4ZD (GB)
(72) Inventor: BRICCHI, Federico, 6708 WH Wageningen (NL); WOODALL, Dawn, Louise, 6708 WH Wageningen (NL)
(74) Representative: Unilever Patent Group
(86) International application number: PCT/EP2023/056976
(87) International publication number: WO 2023/186583

(56) References cited:
- WO-A1-2015/158639
- WO-A1-2017/194901
- US-A1- 2003 124 067
- US-A1- 2012 020 898
- US-A1- 2015 366 767
- US-A1- 2020 261 332

## Description

### Field of the Invention

The present invention is concerned with non-aqueous oral care compositions.

### Background of the Invention

Oral care compositions such as dentifrices typically contain dentally acceptable abrasive, humectant, water, and water-soluble polymer which serves as a thickener and binder for the ingredients. A variety of other ingredients such as flavours, sweeteners, preservatives and fluoride are also utilized at low levels.

However, there are many materials which are physically or chemically incompatible with the aqueous environments found in typical dentifrice formulations.

Non-aqueous formulations have been suggested as a way of improving the stability of these materials. For example, WO96/03108 describes a non-aqueous dentifrice composition comprising a carboxyvinyl polymer, a humectant, a polyethylene glycol and a dentally acceptable abrasive.

EP 2585031 discloses a non-aqueous oral care composition with a liquid continuous phase comprising a thickening agent, a humectant, and one or more liquid polyethylene glycols having a melting point below 25°C, in which the liquid continuous phase is structured with crystals of one or more solid polyethylene glycols having a melting point of 25°C or above. US2012/020898 discloses stable non aqueous oral care compositions comprising carrageenan and glycerin. Said document does not disclose the proportion of iota to kappa carrageenan. US2020/261332 discloses in the examples compositions that comprise more than 50 wt % glycerin. Said compositions comprise a total concentration of carrageenan varying from 0,3 to 0,5 wt %.

However there remains the need for non-aqueous compositions with a good viscosity profile and flow characteristics as well as excellent storage characteristics.

It is thus an object of the present invention to provide for an oral care composition with good paste shape and ribbon height.

### Description of the Invention

A first aspect of the invention relates to a non-aqueous oral care composition comprising:
i) greater than 50 wt% of an organic polyol having 3 or more hydroxyl groups in the molecule;
ii) 0.2 wt % or less of the total composition of carrageenan; which the carrageenan consists of a 1:2 to 2:1 weight ratio of iota to kappa carrageenan.
in which the term non-aqueous no more than 5 wt% of water of the total composition.

### Detailed Description of the Invention

Except in the examples, or where otherwise explicitly indicated, all numbers in this description indicating amounts of material or conditions of reaction, physical properties of materials and/or use may optionally be understood as modified by the word "about".

All amounts are by weight of the final composition, unless otherwise specified.

It should be noted that in specifying any ranges of values, any particular upper value can be associated with any particular lower value.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

Any ingredients mentioned in this application that are natural or naturally derived have been sourced from Europe.

The composition of the invention is non-aqueous. By "non-aqueous" it is generally meant that water is not deliberately added to the composition in any significant quantity. However, the term "non-aqueous" does not mean that small amounts of water cannot be present, for example as a consequence of its association with hygroscopic raw materials. Accordingly, for the purposes of this invention, the term "non-aqueous" means that water is present in an amount no greater than about 5%, more preferably no greater than about 3% most preferably no greater than 1% by weight based on the total weight of the composition.

Compositions according to the invention comprise carrageenan at weight ratios of 1:2 to 2:1 iota to kappa carrageenan. The carrageenan present in the composition of the invention preferably consists from 33wt% to 66wt% of the total level of iota carrageenan and 33 wt% to 66 wt% of kappa carrageenan.

The total level of carrageenan is preferably greater than 0.01 to 1 wt% of the total composition, more preferably greater than 0.05 wt% most preferably greater than 0.08 wt%.

The composition of the invention comprise polyethylene glycol or polypropylene glycol having a melting point below 42°C, more preferably below 25°C. In a preferred embodiment of the invention the melting point is greater than -20°C and ranges from -15 to 25°C, more preferably from -5 to 8°C, most preferably from 4 to 8°C.

Preferred liquid polyethylene glycols have an average value of n in the general formula H(OCH₂CH₂)ₙ OH (as described above) ranging from about 4 to 12, more preferably about 6 to 8, most preferably about 8. The average molecular weight suitably ranges from about 190 to 630, more preferably from about 285 to 420, most preferably from about 380 to 420 g/mol. Suitable commercially available materials include for example PEG 400 (ex BDH Chemicals). Mixtures of any of the above described materials may also be used.

The amount of polyethylene glycol of polyethylene glycol and/or polypropylene glycol iii (as defined above) in compositions of the invention suitably ranges from 0.1 to 10 wt% of the total composition, more preferably 0.5 to 7wt% most preferably 1 to 5 wt% on the total weight of the composition.

The preferred ratio of polyethylene glycol and/or polypropylene glycol iii) to carrageenan is from 1:200 to 1:4, preferably from 1:1 to 1:40.

Compositions of the invention comprise organic polyols having 3 or more hydroxyl groups in the molecule (hereinafter termed "organic polyols"). Examples of such materials include glycerol, sorbitol, xylitol, mannitol, lactitol, maltitol, erythritol, and hydrogenated partially hydrolyzed polysaccharides. The most preferred organic polyol is glycerol. Mixtures of any of the above described materials may also be used.

The level of organic polyol will depend on the particular type chosen, but generally ranges from about 20 to 90% by weight based on the total weight of the composition, the amount of organic polyol suitably ranges from 35 to 85%, more preferably from 65 to 80% by total weight organic polyol based on the total weight of the composition. Ideally the composition of the invention is glycerol-based (i.e. glycerol makes up a higher portion of the composition's weight than any other compound) and contains from 55 to 80% by weight glycerol based on the total weight of the composition.

Compositions of the invention preferably comprise an anionic surfactants, more preferably an at least 50wt% of the total anionic surfactant of an alkyl sulphate based surfactant. Most preferably the anionic surfactant is sodium lauryl sulphate.

Preferably the level of anionic surfactant, more preferably sodium lauryl sulphate is from 0.1 to 5 wt%, preferably 0.5 to 3 wt% of the total composition.

Additional surfactants, such as nonionic, cationic and zwitterionic or amphoteric surfactants are preferably present at a level below 0.5 wt of the total composition, preferably less than 0.1 wt% of the composition.

The composition of the invention is used to preferably used to clean the surfaces of the oral cavity and is known as an oral care composition.

Accordingly, preferred product forms for compositions of the invention are those which are suitable for brushing and/or rinsing the surfaces of the oral cavity.

The composition of the invention is most preferably in the form of a toothpaste. Such a composition is not intentionally swallowed for purposes of systemic administration of therapeutic agents, but is applied to the oral cavity, used to treat the oral cavity and then expectorated. Typically, such a composition is used in conjunction with a cleaning implement such as a toothbrush, usually by applying it to the bristles of the toothbrush and then brushing the accessible surfaces of the oral cavity.

Preferably the dentifrice/toothpaste is in the form of an extrudable semi-solid such as a cream, paste or gel (or mixture thereof).

A composition according to the invention (such as a dentifrice/toothpaste) will generally contain further ingredients to enhance performance and/or consumer acceptability, in addition to the ingredients specified above.

Compositions according to the invention, particularly toothpastes, preferably comprise particulate abrasive materials such as silicas, aluminas, calcium carbonates, dicalcium phosphates, calcium pyrophosphates, hydroxyapatites, trimetaphosphates, insoluble hexametaphosphates and so on, including agglomerated particulate abrasive materials.

Preferably the composition, particularly a toothpaste, comprises a silica based abrasive. The preferred abrasive silicas used in the present invention is a silica with a low refractive index. It may be used as the sole abrasive silica, or in conjunction with a low level of other abrasive silicas, e.g. those according to EP 236070. The low refractive index silicas, used as abrasives in the present invention are preferably silicas with an apparent refractive index (R.I.) in the range of 1.41 - 1.47, preferably 1.435 - 1.445, preferably having a weight mean particle size of between 5 and 15 mm, a BET (nitrogen) surface area of between 10 and 100 m²/g and an oil absorption of about 70 - 150 cm³/100 g, but abrasive silicas with a lower apparent refractive index may also be used. Typical examples of suitable low refractive index abrasive silicas (e.g. having an R.I. of between 1.435 and 1.445) are Tixosil 63 and 73 ex Rhone Poulenc; Sident 10 ex Degussa; Zeodent 113 ex Zeofinn; Zeodent 124 ex Evonik, Sorbosil AC 77 ex PQ Corporation (having an R.I. of approximately 1.440). The amount of these silicas in the composition generally ranges from 5-60% by weight, usually 5-20% by weight.

The composition, particularly if a toothpaste preferably comprises an inorganic or a natural or synthetic thickener or gelling agent in proportions of about 0.10 to about 15% by weight depending on the material chosen. These proportions of thickeners in the dentifrice compositions of the present invention form an extrudable, shape-retaining product which can be squeezed from a tube onto a toothbrush and will not fall between the bristles of the brush but rather, will substantially maintain its shape thereon. Suitable thickeners or gelling agents useful in the practice of the present invention include inorganic thickening silicas such as amorphous silicas available from Huber Corporation under the trade designation Zeodent 165, Irish moss, iota-carrageenan, gum tragacanth, and polyvinylpyrrolidone.

Compositions according to the invention preferably comprise a polymeric deposition aid. Preferably the composition comprises acid anhydride polymers, particularly preferred are co-polymers of maleic anhydride with methyl vinylether, in which the anhydride moiety may be in a partially or fully hydrolysed or alcoholysed form. Preferred copolymers include Gantrez(R) polymers such as:
Gantrez S-95: molecular weight 216,000; free acid;
Gantrez S-96: molecular weight 700,000; free acid;
Gantrez S-97: molecular weight 1,500,000; free acid; and
Gantrez MS-955: molecular weight 1,060,000; calcium/sodium salt.

Particularly preferred co-polymers of maleic acid and methyl vinylether have a molecular weight of 1,000,000 or greater and an especially preferred material is Gantrez S-97.

Compositions according to the invention may comprise a tooth whitening agent. The whitening agent preferably comprises a green and/or a blue pigment. In the context of the present invention a pigment is generally understood to be a shade/material which is insoluble in the relevant medium, at the relevant temperature. This is in contrast to dyes which are soluble. In the context of this invention, the "relevant medium" is human saliva, the liquid medium in which the composition is used, at the temperature of the oral cavity during brushing of the teeth, i.e. up to 37 Degrees C. As a reasonable approximation, the relevant medium may be considered to be water and the relevant temperature to be 25 Degrees C.

Preferably the blue pigment is Pigment Blue 15, more preferably Pigment Blue 15:1, 15:2, 15:3, 15:4, 15:5 or 15:6, most preferably 15:1. A preferred pigment is blue pigment is Phthalocyanine Blue Pigment, Cl No. 74160, blue covarine.

The preferred Green pigment is Phthalocyanine Green, preferably Phthalocyanine Green CI-74260.

Preferably the total level of pigment in the composition is from 0.01 wt% to 3 wt, more preferably from 0.02 to 2 wt%.

If the composition is a toothpaste it may be a dual phase paste, with the whitening pigments present in one phase.

Compositions according to the invention may comprise water-soluble or sparingly water-soluble sources of metal salts Preferred are zinc ions such as zinc chloride, zinc acetate, zinc gluconate, zinc sulphate, zinc fluoride, zinc citrate, zinc lactate, zinc oxide, zinc monoglycerolate, zinc tartrate, zinc pyrophosphate and zinc maleate; also preferred are stannous ions such as stannous fluoride and stannous chloride.

Compositions according to the invention may comprise oral care enzyme systems such as hydrogen peroxide producing enzyme systems (e.g. the oxidoreductase enzyme glucose oxidase), amyloglucosidase, dextranase and/or mutanase, (optionally in the presence of zinc ion providing compounds and/or 8- hydroxyquinoline derivatives), lactoperoxidase, lactoferrin, lysozyme and mixtures thereof.

Compositions of the invention may comprise fluoride sources such as sodium fluoride, stannous fluoride, sodium monofluorophosphate, zinc ammonium fluoride, tin ammonium fluoride, calcium fluoride, cobalt ammonium fluoride and mixtures thereof.

In one embodiment a preferred class of oral care active for inclusion in the compositions of the invention includes agents for the remineralisation of teeth. The term "remineralisation" in the context of the present invention means the *in situ* generation of hydroxyapatite on teeth.

A specific example of a suitable agent for the remineralisation of teeth is a mixture of a calcium source and a phosphate source which, when delivered to the teeth results in the *in situ* generation of hydroxyapatite on teeth.

Illustrative examples of the types of calcium source that may be used in this context (hereinafter termed "remineralising calcium sources") include, for example, calcium phosphate, calcium gluconate, calcium oxide, calcium lactate, calcium glycerophosphate, calcium carbonate, calcium hydroxide, calcium sulphate, calcium carboxymethyl cellulose, calcium alginate, calcium salts of citric acid, calcium silicate and mixtures thereof. Preferably the remineralising calcium source is calcium silicate.

The amount of remineralising calcium source(s) (e.g. calcium silicate) in the composition of the invention typically ranges from 1 to 30%, preferably from 5 to 20% by total weight remineralising calcium source based on the total weight of the oral care composition.

Illustrative examples of the types of phosphate source that may be used in this context (hereinafter termed "remineralising phosphate sources") include, for example, monosodium dihydrogen phosphate, disodium hydrogen phosphate, sodium pyrophosphate, tetrasodium pyrophosphate, sodium tripolyphosphate, sodium hexametaphosphate, potassium dihydrogenphosphate, trisodium phosphate, tripotassium phosphate and mixtures thereof. Preferably the remineralising phosphate source is a mixture of trisodium phosphate and sodium dihydrogen phosphate.

The amount of remineralising phosphate source(s) (e.g. trisodium phosphate and sodium dihydrogen phosphate) in the composition of this invention typically ranges from 2 to 15%, preferably from 4 to 10% by total weight remineralising phosphate source based on the total weight of the oral care composition.

Mixtures of any of the above described materials may also be used.

The composition according to the invention will comprise further ingredients which are common in the art, such as:
antimicrobial agents, e.g. chlorhexidine, sanguinarine extract, metronidazole, quaternary ammonium compounds, such as cetylpyridinium chloride; cetylpyridium chloride clay complex bis-guanides, such as chlorhexidine digluconate, hexetidine, octenidine, alexidine; and halogenated bisphenolic compounds, such as 2,2' methylenebis-(4-chloro-6-bromophenol);
anti-inflammatory agents such as ibuprofen, flurbiprofen, aspirin, indomethacin etc.;
anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C and E;
plant extracts;
plant-derivable antioxidants such as flavonoid, catechin, polyphenol, and tannin compounds and mixtures thereof;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
hyaluronic acid;
amino acids such as arginine;
colouring agents;
pH-adjusting agents;
sweetening agents;
pharmaceutically acceptable carriers, e.g. starch, sucrose, water or water/alcohol systems etc.; Humectants such as glycerol, sorbitol, propylene glycol, xylitol, lactitol etc.;
binders and thickeners such as sodium carboxymethyl-cellulose, hydroxyethyl cellulose (Natrosol^{®}), xanthan gum, gum arabic etc. as well as synthetic polymers such as polyacrylates and carboxyvinyl polymers such as Carbopol^{®};
polymeric compounds which can enhance the delivery of active ingredients such as antimicrobial agents can also be included;
buffers and salts to buffer the pH and ionic strength of the oral care composition; and other optional ingredients that may be included are e.g. bleaching agents such as peroxy compounds e.g. potassium peroxydiphosphate, effervescing systems such as sodium bicarbonate/citric acid systems, colour change systems, and so on.

Example of the invention will now be illustrated by the following examples

### Examples

**Table 1**

| **Ingredient** | **Wt. %** | **Wt. %** | **Wt. %** |
|---|---|---|---|
| | **Example A** | **Example B** | **Example 1** |
| Glycerin | 68.49 | 68.49 | 68.49 |
| Carrageenan Kappa | | 0.0375 | 0.075 |
| Carrageenan Iota | 0.15 | 0.1125 | 0.075 |
| Xanthan Gum | 0.1 | 0.1 | 0.1 |
| Adinol CT95 | 1.5 | 1.5 | 1.5 |
| Trisodium Phosphate anhydrous | 6 | 6 | 6 |
| Monosodium Phosphate anhydrous | 1 | 1 | 1 |
| Calcium silicate | 15 | 15 | 15 |
| Monocalcium phosphate anhydrous | 3.5 | 3.5 | 3.5 |
| Hydrated Low abrasive silica | 2 | 2 | 2 |
| Sodium Monofluorophosphate | 1.11 | 1.11 | 1.11 |
| Minors | To 100 | To 100 | To 100 |
| Viscosity(cP) at 25°C | 361 | 910 | 1258 |
| Ribbon height to width ratio at 25°C | 0.38 | 0.48 | 0.6 |

**Table 2**

| **Ingredient** | **Wt. %** | **Wt. %** |
|---|---|---|
| | **Example 2** | **Example 3** |
| Glycerin and minors | To 100 | To 100 |
| Carrageenan Kappa | 0.1 | 0.08 |
| Carrageenan Iota | 0.1 | 0.08 |
| Empicol LZV/N ^{*1} | 2 | 2 |
| Trisodium Phosphate anhydrous | 3.8 | 3.8 |
| Monosodium Phosphate anhydrous | 3.2 | 3.2 |
| Calcium silicate | 15 | 15 |
| Sodium Monofluorophosphate | 1.11 | 1.11 |

| | | |
|---|---|---|
| *1 sodium lauryl sulfate | | |

## Claims

1. A non-aqueous oral care composition comprising:
i) greater than 50 wt% of an organic polyol having 3 or more hydroxyl groups in the molecule;
ii) 0.2 wt % or less of the total composition of carrageenan; which the carrageenan consists of a 1:2 to 2:1 weight ratio of iota to kappa carrageenan;
in which the term non-aqueous no more than 5 wt% of water of the total composition.

2. A non-aqueous oral care composition further comprising an anionic surfactant, preferably an alkyl sulphate based surfactant more preferably sodium lauryl sulphate.

3. A non-aqueous oral care composition any preceding claim in which the organic polyol is glycerol.

4. A non-aqueous oral care composition according to any preceding claim further comprising polyethylene glycol and/or polypropylene glycol having a melting point below 42°C, preferably below 25°C.

5. A non-aqueous oral care composition according to claim 4 in which the polyethylene glycol having a melting point above -20°C.

6. A non-aqueous oral care composition according to any preceding claim in which the level of carrageenan is greater than 0.01 wt% of the total composition, more preferably greater than 0.05 wt% most preferably greater than 0.08 wt%.

7. A non-aqueous oral care composition according to any preceding claim in which the level of polyethylene glycol and/or polypropylene glycol is from 0.1 to 10 wt% of the total composition, more preferably 0.5 to 7wt% most preferably 1 to 5 wt%.

8. A non-aqueous oral care composition according to any preceding claim in which the weight ratio of polyethylene glycol and/or polypropylene glycol iii) to carrageenan is from 1:200 to 1:4, preferably from 1:1 to 1:40.

9. A non-aqueous oral care composition according to any preceding claim in which the level of polyol is greater than 65wt% of the total composition.

10. A non-aqueous oral care composition according to any preceding claim that is a toothpaste.

11. A non-aqueous oral care composition according to any preceding claim which further comprises an abrasive.

12. A non-aqueous oral care composition according to any preceding claim composition in which the level anionic surfactant, preferably sodium lauryl sulphate is from 0.1 to 5 wt%, preferably 0.5 to 3 wt% of the total composition.

13. A non-aqueous oral care composition according to any preceding claim that further comprises a mixture of a calcium source and a phosphate source which, when delivered to the teeth results in the *in situ* generation of hydroxyapatite on teeth.

14. A non-aqueous composition according to claim 13 in which the calcium source is calcium silicate.

## Patentansprüche

1. Nichtwässrige Mundpflegezusammensetzung, umfassend:
i) mehr als 50 Gew.-% eines organischen Polyols mit 3 oder mehr Hydroxylgruppen im Molekül;
ii) 0,2 Gew.-% oder weniger der Gesamtzusammensetzung an Carrageen; wobei das Carrageen ein Gewichtsverhältnis von Iota- zu Kappa-Carrageen von 1:2 bis 2:1 aufweist;
wobei der Begriff "nichtwässrig" bedeutet, dass höchstens 5 Gew.-% Wasser in der Gesamtzusammensetzung vorliegen.

2. Nichtwässrige Mundpflegezusammensetzung, die ferner ein anionisches Tensid, vorzugsweise ein Tensid auf Alkylsulfatbasis, bevorzugter Natriumlaurylsulfat, umfasst.

3. Nichtwässrige Mundpflegezusammensetzung nach einem vorhergehenden Anspruch, wobei das organische Polyol Glycerin ist.

4. Nichtwässrige Mundpflegezusammensetzung nach einem vorhergehenden Anspruch, die ferner Polyethylenglykol und/oder Polypropylenglykol mit einem Schmelzpunkt unter 42°C, vorzugsweise unter 25°C, umfasst.

5. Nichtwässrige Mundpflegezusammensetzung nach Anspruch 4, wobei das Polyethylenglykol einen Schmelzpunkt über -20°C aufweist.

6. Nichtwässrige Mundpflegezusammensetzung nach einem vorhergehenden Anspruch, wobei der Gehalt an Carrageen größer als 0,01 Gew.-% der Gesamtzusammensetzung, bevorzugter größer als 0,05 Gew.-% und höchst bevorzugt größer als 0,08 Gew.-% ist.

7. Nichtwässrige Mundpflegezusammensetzung nach einem vorhergehenden Anspruch, wobei der Gehalt an Polyethylenglykol und/oder Polypropylenglykol 0,1 bis 10 Gew.-% der Gesamtzusammensetzung beträgt, bevorzugter 0,5 bis 7 Gew.- % und höchst bevorzugt 1 bis 5 Gew.-%.

8. Nichtwässrige Mundpflegezusammensetzung nach einem vorhergehenden Anspruch, wobei das Gewichtsverhältnis von Polyethylenglykol und/oder Polypropylenglykol iii) zu Carrageen 1:200 bis 1:4, vorzugsweise 1:1 bis 1:40 beträgt.

9. Nichtwässrige Mundpflegezusammensetzung nach einem vorhergehenden Anspruch, wobei der Gehalt an Polyol größer als 65 Gew.-% der Gesamtzusammensetzung beträgt.

10. Nichtwässrige Mundpflegezusammensetzung nach einem vorhergehenden Anspruch, die eine Zahnpasta darstellt.

11. Nichtwässrige Mundpflegezusammensetzung nach einem vorhergehenden Anspruch, die ferner ein Schleifmittel enthält.

12. Nichtwässrige Mundpflegezusammensetzung nach einem vorhergehenden Anspruch, wobei der Gehalt an anionischem Tensid, vorzugsweise Natriumlaurylsulfat, 0,1 bis 5 Gew.-%, vorzugsweise 0,5 bis 3 Gew.-% der Gesamtzusammensetzung beträgt.

13. Nichtwässrige Mundpflegezusammensetzung nach einem vorhergehenden Anspruch, die ferner eine Mischung aus einer Calciumquelle und einer Phosphatquelle umfasst, die nach dem Aufbringen auf die Zähne zur in-situ-Bildung von Hydroxyapatit auf den Zähnen führt.

14. Nichtwässrige Zusammensetzung nach Anspruch 13, wobei die Calciumquelle Calciumsilikat ist.

## Revendications

1. Composition de soin buccodentaire non aqueuse comprenant :
i) plus de 50 % en poids d'un polyol organique ayant 3 ou plus de 3 groupes hydroxyle par molécule ;
ii) 0,2 % ou moins, en poids de la composition totale, de carraghénane ; laquelle carraghénane consiste en un rapport des carraghénanes iota à kappa de 1/2 à 2/1 en poids ;
dans laquelle l'expression "non aqueuse" signifie qu'il y a au plus 5 % en poids d'eau dans la composition totale.

2. Composition de soin buccodentaire non aqueuse comprenant en outre un tensioactif anionique, de préférence un tensioactif à base d'alkylsulfate, mieux encore le laurylsulfate de sodium.

3. Composition de soin buccodentaire non aqueuse selon l'une quelconque des revendications précédentes, dans laquelle le polyol organique est le glycérol.

4. Composition de soin buccodentaire non aqueuse selon l'une quelconque des revendications précédentes, comprenant en outre un polyéthylèneglycol et/ou polypropylèneglycol ayant un point de fusion inférieur à 42 °C, de préférence inférieur à 25 °C.

5. Composition de soin buccodentaire non aqueuse selon la revendication 4, dans laquelle le polyéthylèneglycol a un point de fusion supérieur à -20 °C.

6. Composition de soin buccodentaire non aqueuse selon l'une quelconque des revendications précédentes, dans laquelle le niveau de carraghénane est supérieur à 0,01 % en poids de la composition totale, mieux encore supérieur à 0,05 % en poids, tout spécialement supérieur à 0,08 % en poids.

7. Composition de soin buccodentaire non aqueuse selon l'une quelconque des revendications précédentes, dans laquelle le niveau de polyéthylèneglycol et/ou polypropylèneglycol est de 0,1 à 10 % en poids de la composition totale, mieux encore 0,5 à 7 % en poids, tout spécialement 1 à 5 % en poids.

8. Composition de soin buccodentaire non aqueuse selon l'une quelconque des revendications précédentes, dans laquelle le rapport en poids du polyéthylèneglycol et/ou polypropylèneglycol iii) à la carraghénane est de 1/200 à 1/4, de préférence de 1/1 à 1/40.

9. Composition de soin buccodentaire non aqueuse selon l'une quelconque des revendications précédentes, dans laquelle le niveau de polyol est supérieur à 65 % en poids de la composition totale.

10. Composition de soin buccodentaire non aqueuse selon l'une quelconque des revendications précédentes, qui est une pâte dentifrice.

11. Composition de soin buccodentaire non aqueuse selon l'une quelconque des revendications précédentes, qui comprend en outre un abrasif.

12. Composition de soin buccodentaire non aqueuse selon l'une quelconque des revendications précédentes, dans laquelle le niveau de tensioactif anionique, de préférence de laurylsulfate de sodium, est de 0,1 à 5 % en poids, de préférence de 0,5 à 3 % en poids de la composition totale.

13. Composition de soin buccodentaire non aqueuse selon l'une quelconque des revendications précédentes, qui comprend en outre un mélange d'une source de calcium et d'une source de phosphate qui, lorsqu'il est délivré aux dents, a pour résultat la génération *in situ* d'hydroxyapatite sur les dents.

14. Composition de soin buccodentaire non aqueuse selon la revendication 13, dans laquelle la source de calcium est le silicate de calcium.
